(19) Europäisches Patentamt
European Patent Office
Office européen des brevets

(11) EP 4 782 834 A1

(12) EUROPEAN PATENT APPLICATION
published in accordance with Art. 153(4) EPC

(43) Date of publication:
29.07.2026 Bulletin 2026/31

(21) Application number: 24868048.0

(22) Date of filing: 27.08.2024

(51) International Patent Classification (IPC):
*G01N 33/49* (2006.01)  *G01N 35/00* (2006.01)

(52) Cooperative Patent Classification (CPC):
G01N 33/49; G01N 35/00

(86) International application number:
PCT/JP2024/030482

(87) International publication number:
WO 2025/062968 (27.03.2025 Gazette 2025/13)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 22.09.2023 JP 2023158397

(71) Applicant: HITACHI HIGH-TECH CORPORATION
Tokyo 105-6409 (JP)

(72) Inventors:
• KAKISHITA Yasuki
Tokyo 100-8280 (JP)
• HATTORI Hideharu
Tokyo 100-8280 (JP)
• TAMEZANE Hideto
Tokyo 105-6409 (JP)
• MUKAIYAMA Naoki
Tokyo 105-6409 (JP)

(74) Representative: Strehl & Partner mbB
Maximilianstrasse 54
80538 München (DE)

(54) **SAMPLE STATE DETERMINATION DEVICE, SAMPLE STATE DETERMINATION METHOD, AND SAMPLE ANALYSIS SYSTEM**

(57)    A specimen state judging apparatus includes: an image input unit that receives an image obtained by imaging the vessel storing the specimen from the side surface of the vessel; a region detection unit that identifies a blood serum or blood plasma region from within the image; a state judgment properness information calculation unit that calculates state judgment properness information as information for evaluating a properness degree intended for the state judgment of the specimen for each partial region of the blood serum or blood plasma region identified by the region detection unit; a state judgment region selection unit that selects the partial region suitable for the state judgment of the specimen on the basis of the state judgment properness information; a state judgment unit that judges the state of the specimen by using the partial region selected by the state judgment region selection unit; and an output unit that outputs the state judgment result of the specimen judged by the state judgment unit and/or position information within the image of the partial region selected by the state judgment region selection unit.

FIG. 2

SAMPLE IMAGE — 101

201 IMAGE INPUT UNIT

202 BLOOD SERUM (BLOOD PLASMA) REGION DETECTION UNIT

203 STATE JUDGMENT PROPERNESS INFORMATION CALCULATION UNIT

204 STATE JUDGMENT REGION SELECTION UNIT

205 STATE JUDGMENT UNIT

206 OUTPUT UNIT

STATE JUDGMENT RESULT
STATE JUDGMENT REGION SELECTION RESULT

Processed by Luminess, 75001 PARIS (FR)

EP 4 782 834 A1

**Description**

Incorporation by Reference

The present application claims priority from Japanese patent application 2023-158397 filed on September 22, 2023, the content of which is hereby incorporated by reference into this application.

Technical Field

The present invention relates to a technique for judging a state of a specimen applicable to an automatic blood analyzer, such as a biochemical analyzer and an immune automatic analyzer.

Background Art

An automatic blood analyzer, such as a biochemical analyzer and an immune analyzer performs analysis on the basis of coloring and luminescence caused from a reaction solution made by reacting a specimen with a reagent. When at this time, the specimen contains the lipemia (L), the hemolysis (H), the icterus (I), and the like, this may affect the accuracy properties of the analysis result.

The operation of visually selecting a sample containing such the specimen has a large load on the user, and has high needs for automation. For example, Patent Literature 1 discloses a method by which a plurality of images of a sample vessel including the blood serum or blood plasma portion of a sample are acquired, the lipemia properties, the hemolytic properties, and the icteric properties of the blood serum or blood plasma region are judged for each pixel or for each patch from the plurality of images by using a convolutional neural network, and the results are integrated based on majority rule and the like within the blood serum or blood plasma region, thereby judging the LHI of the sample.

Citation List

Patent Literature

Patent Literature 1: Japanese Unexamined Patent Application Publication (Translation of PCT Application) No. 2020-519853

Summary of Invention

Technical Problem

However, the vessel storing the specimen and the state of the inside of the specimen are various, and the color tone and the brightness value on the image may not be uniform even within the blood serum or blood plasma region of the same sample. For example, the structural object containing the separating agent contained in the test tube, the label and the seal stuck on the test tube, and the printing and the like on the test tube may affect the color tone and the brightness value of the blood serum or the blood plasma on the image, and shield the blood serum or blood plasma region.

In addition, in the blood serum or the blood plasma, when the illumination illuminates the specimen from above the sample for example, the upper side of the blood serum or the blood plasma becomes bright, and the lower side of the blood serum or the blood plasma becomes dark, so that the brightness value of the blood serum or blood plasma region may become non-uniform. In this way, from the reasons that for example, the color tone and the brightness value of the blood serum or blood plasma region are changed and are non-uniform due to the factor other than the blood serum or the blood plasma, and the blood serum or blood plasma region is shielded, the region that is not suitable for the judgment of the state (such as the LHI) of the specimen may be included in the blood serum or blood plasma region. When the image of such the blood serum or blood plasma region is used for judging the state of the sample, the conventional method may adversely affect the judgment result of the state of the sample.

Accordingly, an object of the present invention is to provide an apparatus and a method for judging the state of a specimen with high accuracy.

Solution to Problem

A specimen state judging apparatus according to one aspect of the present invention includes: an image input unit that receives an image obtained by imaging the vessel storing the specimen from the side surface of the vessel; a region detection unit that identifies a blood serum or blood plasma region from within the image; a state judgment properness information calculation unit that calculates state judgment properness information as information for evaluating a properness degree intended for the state judgment of the specimen for each partial region of the blood serum or blood plasma region identified by the region detection unit; a state judgment region selection unit that selects the partial region suitable for the state judgment of the specimen on the basis of the state judgment properness information; a state judgment unit that judges the state of the specimen by using the partial region selected by the state judgment region selection unit; and an output unit that outputs the state judgment result of the specimen judged by the state judgment unit and/or position information within the image of the partial region selected by the state judgment region selection unit.

Advantageous Effects of Invention

According to the specimen state judging apparatus of the one aspect of the present invention, it is possible to judge a state of a specimen with high accuracy.

Brief Description of Drawings

Fig. 1 is a diagram illustrating an example of a hardware configuration of a specimen state judging apparatus according to a first embodiment.
Fig. 2 is a diagram illustrating an example of a functional block diagram of the specimen state judging apparatus according to the first embodiment.
Fig. 3A is a diagram illustrating an example of the detection of upper and lower ends of a blood serum according to the first embodiment.
Fig. 3B is a diagram illustrating an example of the detection of the upper and lower ends of the blood serum according to the first embodiment.
Fig. 4A is a diagram illustrating an example of state judgment properness information according to the first embodiment.
Fig. 4B is a diagram illustrating an example of state judgment properness information according to the first embodiment.
Fig. 5 is a flowchart illustrating an example of a method for selecting a state judgment region according to the first embodiment.
Fig. 6 is a diagram illustrating an example of screen display according to the first embodiment.
Fig. 7 is a diagram illustrating an example of a process flow according to the first embodiment.
Fig. 8 is a diagram illustrating an example of a candidate map and a state judgment intended region according to the first embodiment.
Fig. 9 is a diagram illustrating an example of a system configuration diagram of a specimen analyzing system according to a second embodiment.
Fig. 10 is a diagram illustrating an example of the state judgment properness information according to the first embodiment.

Description of Embodiments

Hereinbelow, embodiments of a specimen state judging apparatus, a specimen state judging method, and a specimen analyzing system according to the present invention will be described with reference to the accompanying drawings. It should be noted that in the following description and the accompanying drawings, the components having the same functional configurations are indicated by the same reference numerals, and the overlapped description thereof is omitted.

First Embodiment

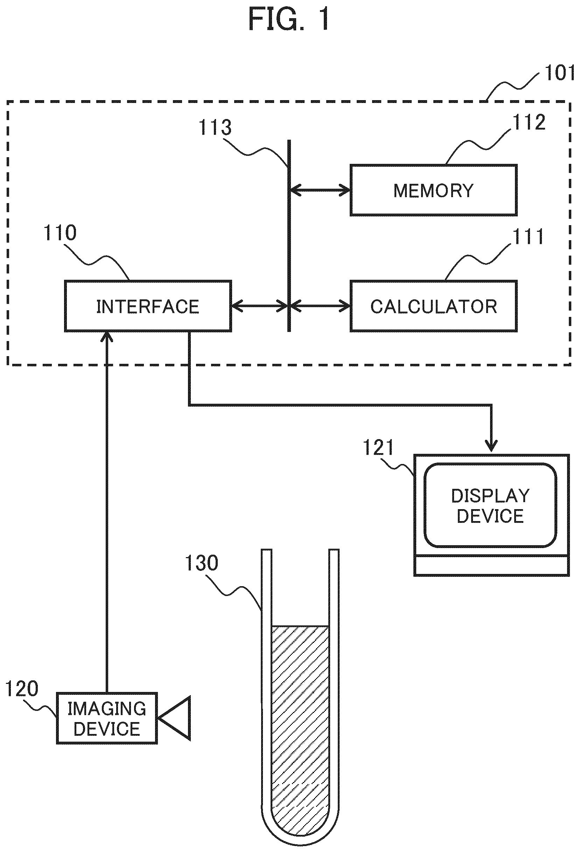

An example of an apparatus that judges the specimen state within an image according to a first embodiment will be described with reference to Fig. 1. Fig. 1 illustrates the hardware configuration example of a specimen state judging apparatus 101 and its peripheral devices.
The specimen state judging apparatus 101 includes an interface 110, a calculator 111, a memory 112, and a bus 113. The interface 110, the calculator 111, and the memory 112 transmit and receive information via the bus 113. In addition, the specimen state judging apparatus 101 transmits and receives a signal between the specimen state judging apparatus 101 and an imaging device 120 and a display device 121 via the interface 110.
Each portion of the specimen state judging apparatus 101 will be described.

The interface 110 is a communication device that transmits and receives a signal between the interface 110 and the devices outside the specimen state judging apparatus 101. As the devices that communicate with the interface 110, the imaging device 120 and the display device 121 are present. The details of the imaging device 120 and the display device 121 will be described later.

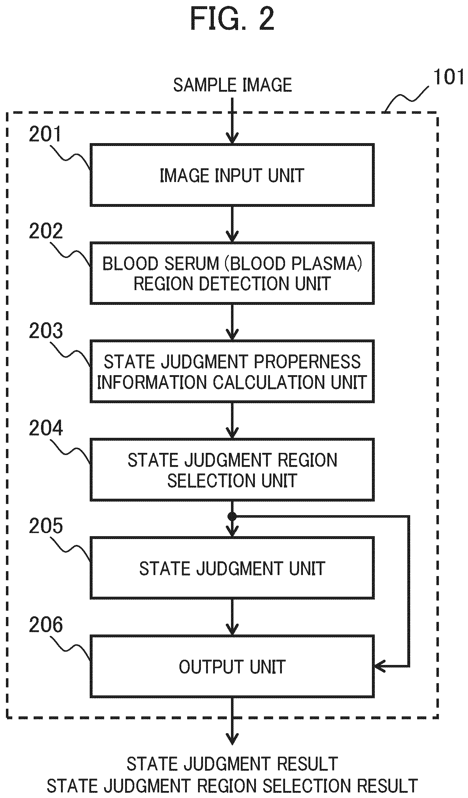

The calculator 111 is a device that executes various processes in the specimen state judging apparatus 101, and is, for example, a CPU (Central Processing Unit), an FPGA (Field-Programmable Gate Array), and the like. The functions executed by the calculator 111 will be described later with reference to Fig. 2.

The memory 112 is a device that stores a program executed by the calculator 111, a parameter, a coefficient, a processing result, and the like, and is an HDD, an SSD, a RAM, a ROM, a flash memory, and the like.

The imaging device 120 is a device that images a sample 130 from the side surface of the sample 130, and is, for example, a visible light camera, a hyperspectral camera, and the like. The imaging device 120 transmits the captured image to the specimen state judging apparatus 101. It should be noted that the imaging device 120 may be replaced with a communication device that receives the image via a network and the like, a recording device that reads the image recorded onto a recording medium to receive the image, and the like.

The display device 121 is a device for displaying the specimen state judgment result outputted by the specimen state judging apparatus 101 and/or the position information of the pixel or the region within the image used for judging the specimen state, and is, for example, a display, a printer, and the like.

Hereinafter, the specimen state judging apparatus 101 will be described in detail.

Fig. 2 is an example of the functional block diagram of the specimen state judging apparatus 101 according to the first embodiment. It should be noted that these functions can be achieved by a calculation device, and each function in the calculation device may be configured of dedicated hardware, and may be configured of the calculator 111 and software operated on the calculator 111.

The specimen state judging apparatus 101 includes an image input unit 201, a blood serum (blood plasma) region detection unit 202, a state judgment properness information calculation unit 203, a state judgment region selection unit 204, a state judgment unit 205, and an output unit 206. Hereinbelow, each unit will be described.

The image input unit 201 receives the image obtained by imaging the sample inputted from the interface 110 to be state judged from the side surface of the sample.

The blood serum (blood plasma) region detection unit 202 will be described.

The blood serum (blood plasma) region detection unit 202 detects the blood serum or blood plasma region from within the sample side surface image outputted from the image input unit 201. As a method for detecting the blood serum or blood plasma region, there is, for example, a method by which in the inputted sample side surface image, the upper end and the lower end of the blood serum or blood plasma region are detected. As the detector, a method belonging to machine learning, such as a YOLO and an SSD may be used. Alternatively, the upper end and the lower end of the blood serum or blood plasma region may be detected by the detector by manual designing such as hough transform by making use of a manually designed feature amount, or by the machine learning.

In addition, as another example of the method for detecting the blood serum or blood plasma region, there is region division (semantic segmentation). The region division is a method by which the image is divided into semantical sets for each pixel or for each small region within the image, and for example, the image is divided into the blood serum or blood plasma region, the separating agent region, the blood clot region, the label region, and the like. As the region dividing device, a method belonging to machine learning, such as a U-Net and a SegNet may be used, and a Watershed method and the like may be used to perform the region division.

Typically, to perform the high accuracy region division, a long calculation time is required, which causes the throughput of the analysis to be lowered, so that the present invention will be described by taking, as an example, the method for detecting the upper and lower ends of the blood serum or the blood plasma. It should be noted that in the case of the machine learning method, the detector is trained by a large number of combinations of the input images and the information on the correct upper and lower end positions of the blood serum or the blood plasma, and the blood serum (blood plasma) region detection unit 202 uses the trained (learned) detector.

The blood serum (blood plasma) region detection unit 202 detects the upper and lower ends of the blood serum or the blood plasma by the blood serum or blood plasma upper and lower end detector, and for example, the partial image from the blood serum or blood plasma upper end to the blood serum or blood plasma lower end is extracted to output the partial image as the blood serum or blood plasma region image.

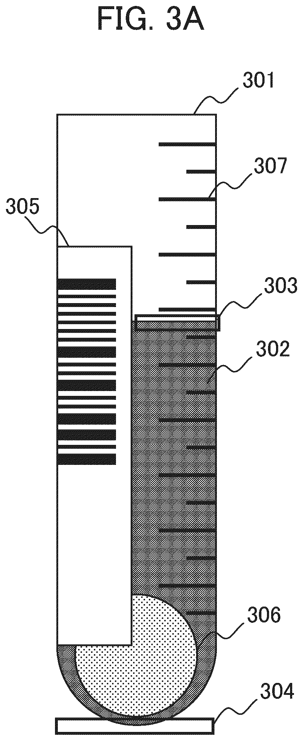

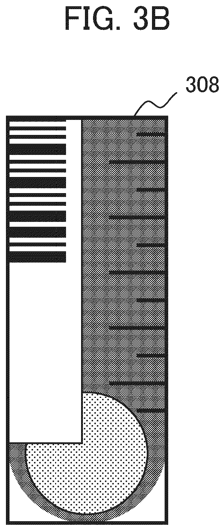

Figs. 3A and 3B illustrate the upper and lower end detection example of the blood serum. Fig. 3A is an example in which a blood serum 302 is stored in a vessel 301. A blood serum upper end detection result 303 and a blood serum lower end detection result 304 represent the upper and lower ends of the blood serum detected by the blood serum (blood plasma) upper and lower end detector. A label 305 is a label attached to the vessel 301, and describes a barcode and sample information. A structural object 306 is an example of a structural object in which the separating agent is stored, and a scale 307 is a scale that is directly printed on the test tube.

Fig. 3B illustrates a blood serum region image 308. The blood serum region 308 is an example of the partial image of the blood serum region extracted on the basis of the blood serum upper end detection result 303 and the blood serum lower end detection result 304. Here, the blood serum region image 308 represents an example in which when the center coordinate in the vertical direction of the blood serum upper end detection result 303 is y1, the center coordinate in the vertical direction of the blood serum lower end detection result 304 is y2, the left end of the blood serum upper end detection result 303 and the blood serum lower end detection result 304 is x1, and the right end of the blood serum upper end detection result 303 and the blood serum lower end detection result 304 is x2, the region having a height y2-y1 and a width x2-x1 is extracted from the start point coordinate (x1, y1). In this way, the blood serum (blood plasma) region detection unit 202 detects the blood serum or blood plasma region to output the blood serum or blood plasma region image.

The state judgment properness information calculation unit 203 will be described.

The state judgment properness information calculation unit 203 calculates the information for each pixel or for each small region for selecting the region intended for the state judgment from within the blood serum or blood plasma region image outputted by the blood serum (blood plasma) region detection unit 202. This information is called state judgment properness information in this specification. The state judgment properness information calculation unit 203 may calculate and output the plurality of pieces of state judgment properness information.

As the example of the state judgment properness information, edge information, color properness degree information, and distance information will be described.

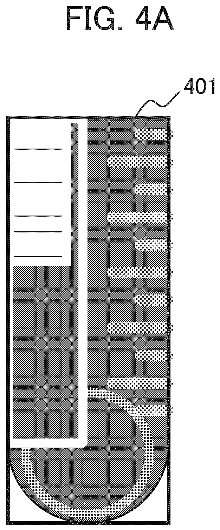

The edge information is information that represents the change in the pixel value (the brightness value, the RGB value, and the like) in the small region within the image. For example, the edge information can be calculated by using a Sobel filter, a local distribution value, and the like. Fig. 4A illustrates the extraction example of the edge information. Edge information 401 represents an example in which the edge of the blood serum region image 308 of Fig. 3B is extracted. As the edge information 401 is closer to white, this represents that the edge component is contained in larger amount.

The edge information 401 includes the strong edge component at a boundary of the label 305 and the barcode, the scale 307, and near a boundary of the structural object 306. These regions may shield the blood serum or blood plasma region and change the color tone and the brightness value of the blood serum or blood plasma region, and thus, are not suitable as the region intended for the state judgment.

By using the edge information, these regions can be excepted from the candidate region intended for the state judgment. The specific excepting method will be described in the state judgment region selection unit 204. It should be noted that in order to detect the region in which the edge component is contained in large amount and its peripheral region, the edge component may be enlarged by dilatation, a filter process, and the like.

The color properness degree information is information that represents how much each pixel or each small region within the blood serum or blood plasma region image is suitable for the color and/or brightness properness range.

As a method for calculating the color properness degree information, there are, for example, a method by which the suitable blood serum or blood plasma image is previously collected (calibration data) and the histogram of the color at each pixel in the calibration data is calculated, a method by which the range of the suitable color range is previously defined on each axis or space of the colorimetric system, such as an RGB, an HSV, and an HLS, and the like.

Here, the present invention will be described by taking, as an example, a method by which the color histogram is used. The example of the calculation equation of the color properness degree information is expressed by Equation (1). Here, the color (x, y) represents the color at the coordinate (x, y) within the blood serum or blood plasma region image, and the HIST(c) is a function that outputs the frequency of the color c in the histogram of the previously calculated calibration data. According to Equation (1), the color properness degree information CA represents the color properness degree, has a high value with respect to the color that appears in the calibration data at high frequency, and has a low value with respect to the color that appears in the calibration data at low frequency.

$$CA(x, y) = HIST(color (x, y))   (1)$$

The color properness degree information calculated in this way is effective for identifying the blood serum or blood plasma-like region. For example, the label and the printing region often has a color different from the color of the blood serum or the blood plasma, so that the color properness degree has a low value. In addition, for example, in the region in which the brightness is excessively increased since the blood serum or the blood plasma is too close to the illumination, the region in which the brightness is excessively decreased since the blood serum or the blood plasma is too far from the illumination, the region in which the color tone and the brightness value of the blood serum or blood plasma region are changed due to the foreign substance in the test tube, and the like, the color properness degree tends to be lowered.

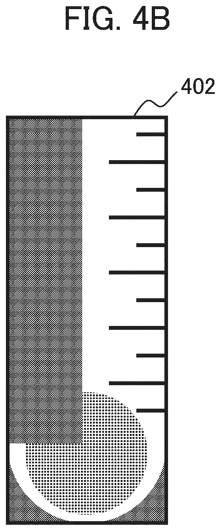

Fig. 4B illustrates the example of the color properness degree information. Color properness degree information 402 represents the calculation example of the color properness degree information of the blood serum region image 308 of Fig. 3B. The color properness degree information 402 represents that as the color properness degree information 402 is closer to white, the color properness degree is higher, and as the color properness degree information 402 is closer to black, the color properness degree is lower. For example, the label 305, the scale 307, the background region, and the like have a very low color properness degree, and are indicated in black. In addition, the region in which the structural object 306 is included is gray since the color tone, the brightness value, and the like of the blood serum on the image are changed. In this way, the color properness degree information is effective for selecting the suitable candidate region intended for the state judgment for the case where the color and the brightness value are varied in the blood serum or the blood plasma in the state judgment region selection unit 204 described later.

Next, the distance information will be described. The distance information is, for example, information that defines the reference point of the blood serum upper end and lower end within the image or the illumination position and the like, and represents how far each pixel is from the reference point. For example, when the sample is illuminated from the upper side, the distance information tends to be brighter toward the blood serum upper end (the sample is closer to the illumination), and the distance information tends to be darker toward the blood serum lower end (the sample is farther from the illumination).

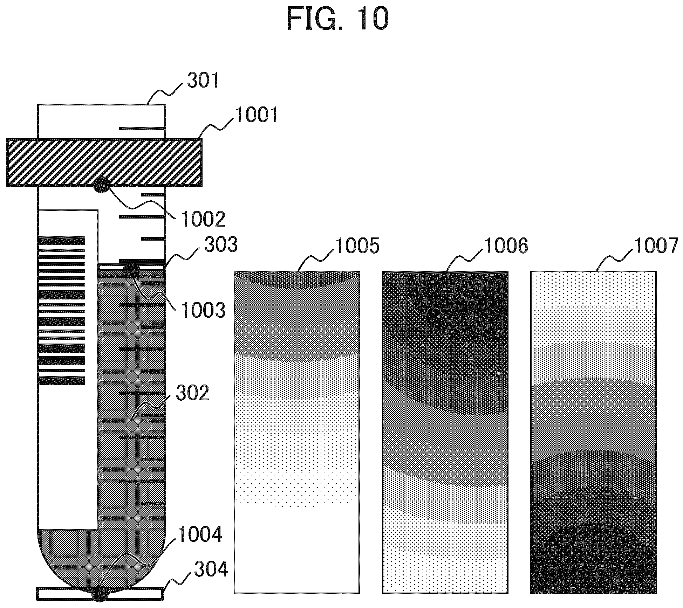

In this way, since there is a relationship between the position within the image and the color tone and the brightness value of the image, the distance information may be outputted as one piece of state judgment properness information in the state judgment properness information. Fig. 10 illustrates the example of the distance information. Fig. 10 illustrates the example in which the blood serum 302 is stored in the vessel 301, and the illumination 1001 illuminations the blood serum 302 in the downward direction. It should be noted that the illumination 1001 represents the example of the illumination position, and the illumination may be set to a different position. In addition, the reference point of the distance is not limited to the illumination, and for example, may be the image upper end and lower end, and the like, and the region and the point detected from within the image, such as the upper end of the blood serum and the vessel may be the reference point. In addition, like Fig. 3A, as the blood serum upper end and lower end, the blood serum upper end result 303 and the blood serum lower end result 304 are assumed to be detected.

An origin point 1002, an origin point 1003, and an origin point 1004 represent the example of the origin point (reference point) at the time of calculating the distance information, the origin point 1002 represents the point closest to the illumination 1001, the origin point 1003 represents the center point of the blood serum upper end result 303, and the origin point 1004 represents the center point of the blood serum lower end 304. Distance information 1005, distance information 1006, and distance information 1007 represent the example of the distance information for the case where the origin point 1002, the origin point 1003, and the origin point 1004 are used as the origin point at the time of calculating the distance information, and the example represents that as the distance information is closer to black, the distance is shorter, and as the distance information is closer to white, the distance is longer.

In this way, the state judgment properness information calculation unit 203 may calculate, as the state judgment properness information, the distance from the origin point to each pixel or each small region for the case where any point within the image is the origin point. It should be noted that the distance from some reference line, such as for example, the horizontal line of the blood serum upper end/lower end, not the point, to each pixel or each small region may be calculated.

The state judgment region selection unit 204 will be described.

The state judgment region selection unit 204 decides and outputs one or more pixels or regions in order for the state judgment unit 205 described later to judge the state by using the state judgment properness information (group) calculated by the state judgment properness information calculation unit 203. Here, the present invention will be described by taking, as an example, the method for selecting the state judgment region using the edge information, the color properness degree information, and the distance information described in the state judgment properness information calculation unit 203.

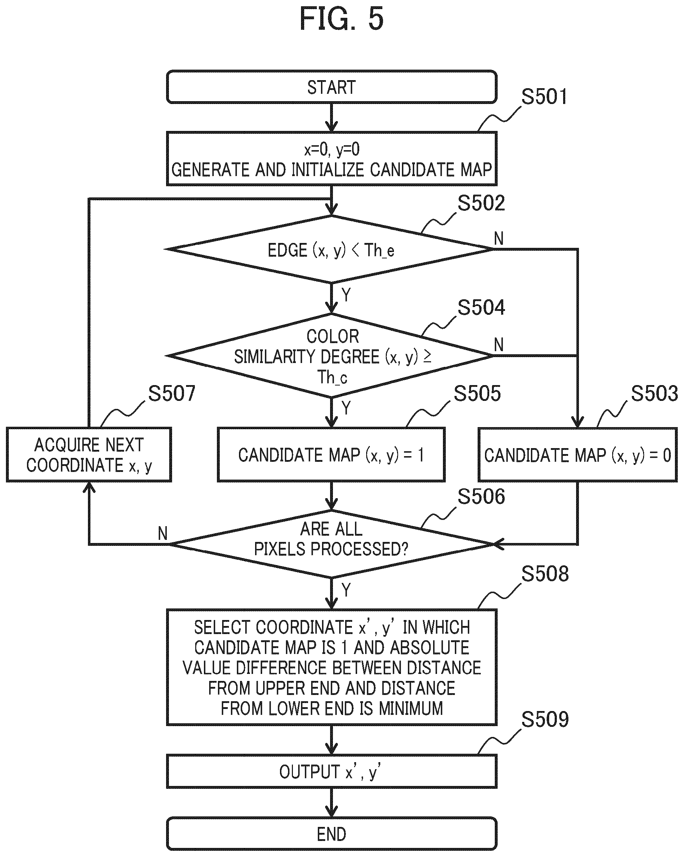

Fig. 5 illustrates a flowchart of an example of the method for selecting the state judgment region. In the example illustrated in Fig. 5, first, the candidate of the state judgment region is selected for each pixel by using the edge information and the color properness degree information, and the distance information is used for deciding the region intended for the final state judgment. It should be noted that only a portion of each of the edge information, the color properness degree information, and the distance information may be referred, and only one kind of information or two kinds of information among these may be referred. For example, only the color properness degree information, or the color properness degree information and the edge information or the distance information may be referred. The more accurate judgment is enabled by increasing the number of kinds of information referred.

In step S501, x and y that are variables representing the coordinate of the pixel or the small region to be selected are initialized to 0, and the candidate map is generated and initialized. The pixel or the small region is the partial region that is the region of a portion of the image of the blood serum region image 308. The candidate map is temporary information for deciding the region intended for the state judgment, and has a two-dimensional array that records whether or not the pixel or the small region is suitable as the candidate of the region intended for the state judgment. Here, the candidate map has the two-dimensional array for the binary value (0 or 1) with the same width and height as the blood serum or blood plasma region image, 1 is stored for the pixel or the region that is judged to be suitable as the region intended for the state judgment, and 0 is stored for the pixel or the region that is judged to be unsuitable. As the initialization, all the values are set to 0.

In step S502, it is judged whether or not the edge information at the coordinate (x, y) is less than the predetermined threshold value Th_e. If true (S502: Y), the flow changes to step S504, and if false (S502:N), the flow changes to step S503. In this step, as described in the state judgment properness information calculation unit 203, the possibility that the region in which the edge component is large and its peripheral region are affected by the label, the printing, the foreign substance, and the like, is high, so that such the region is excepted from the candidate of the region intended for the state judgment.

In step S503, the value of the candidate map at the coordinate (x, y) is set to 0.

In step S504, it is judged whether or not the color properness degree information at the coordinate (x, y) is the predetermined threshold value Th_c or more. If true (S504: Y), the flow shifts to step S505, and if false (S504: N), the flow shifts to step S503. In this step, only the pixel or the small region in which the color properness degree information has the certain value or more is extracted as the region or the pixel suitable for the state judgment.

In step S505, the value of the candidate map at the coordinate (x, y) is set to 1.

In step S506, it is judged whether all the pixels or all the small regions within the blood serum or blood plasma region image are processed. If true (S506: Y), the flow shifts to step S508. If false (S506: N), the flow shifts to step S507.

In step S507, the coordinate of the pixel or the small region to be selected next is acquired. The coordinate should be acquired by using a method by which for example, scanning is performed from the upper left to the lower right of the image. When the new coordinate is acquired, the flow returns to step S502, and the process is repeated until step S506 is true.

In step S508, the region intended for the final state judgment is decided. In step S509, the coordinate of the selected region is outputted. At the timing at which step S508 is executed, the selection of the candidate for each pixel has already been completed, thereby completing the candidate map. Here, as an example, an example in which the region intended for the final state judgment is decided by using the distance information will be described.

First, as the distance information, the information on the distance from the blood serum or blood plasma upper end and the information on the distance from the blood serum or blood plasma lower end are assumed to be inputted. As previously described, in the example of the illumination position illustrated in Fig. 10, the distance information tends to be brighter toward the blood serum upper end, and the distance information tends to be darker toward the blood serum lower end, so that in the candidate region, the region close to the middle between the blood serum upper end and the blood serum lower end is selected as the region intended for the final state judgment. In this way, the region close to the middle between the position closest to the illumination position and the position farthest from the illumination position can be selected as the region intended for the final state judgment.

Accordingly, in this step, the pixel or the small region within the blood serum or blood plasma region image in which the candidate map is 1, and by calculating the absolute value difference between the distance from the blood serum or blood plasma upper end and the distance from the blood serum or blood plasma lower end, the pixel or the small region within the blood serum or blood plasma region image corresponding to the coordinate in which the absolute value difference value is minimum is selected as the pixel or the small region intended for the final state judgment.

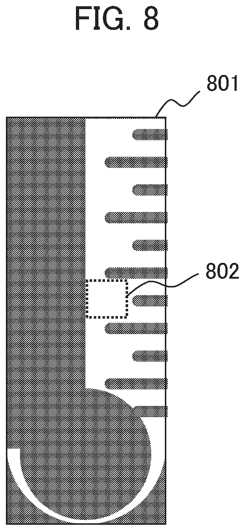

Fig. 8 illustrates the example of the candidate map and the region intended for the state judgment. A candidate map 801 is the example of the candidate map acquired by inputting the edge information 401 and the color properness degree information 402 described in Figs. 4A and 4B to the flow of Fig. 5. In the candidate map 801, black indicates 0, and white indicates 1. The edge periphery and the region in which the color properness degree is low are excepted (black), and only the blood serum region suitable for the state judgment is extracted as the candidate (white). A state judgment intended region 802 is the example of the region intended for the state judgment selected according to the procedure of S508. The state judgment intended region 802 within the blood serum or blood plasma region image is outputted as the region intended for the final state judgment.

In this way, the state judgment region selection unit 204 uses the state judgment properness information (group) calculated by the state judgment properness information calculation unit 203 to select and output the pixel or the small region (partial region) intended for the state judgment. As described above, the state judgment properness information calculation unit 203 may calculate new information by processing or combining the state judgment properness information in order to select the pixel or the small region intended for the state judgment. In addition, for example, in step S508, a plurality of pixels and regions may be extracted in such a manner that the candidate map is 1 and the region closest to the blood serum upper end, the region closest to the blood serum lower end, the region in the middle between them are extracted.

The flow illustrated in Fig. 5 is only an example, and the present invention does not necessarily follow the flow of this flow. The point in which one or more pixels or regions intended for the state judgment are selected by using the state judgment properness information (group) is important, and the selection process is not limited to the example exemplified here.

The state judgment unit 205 will be described.

The state judgment is performed by using the image of the region intended for the state judgment selected by the state judgment region selection unit 204. A state judging device is, for example, a machine learning model, such as a Neural Network, an SVM, and a decision tree. The image 802 of the partial region intended for the state judgment is inputted to the machine learning model. Alternatively, the judgment may be performed by the manual designing in which the manually designed feature amount is extracted from the image 802 of the region intended for the state judgment, thereby using the extracted feature amount, or may be performed by the machine learning model.

The output of the state judging device may be a result obtained by estimating, as a real number value, each index of the LHI, may be a level in which the degree of the LHI is divided into rougher stages, or may be a binary value that represents whether or not the LHI exceeds a predetermined threshold value (Positive or Negative). It should be noted that in the case of the machine learning method, the state judging device is trained by a large number of combinations of the input image and the correct information of the specimen state, and the state judgment region selection unit 204 uses the trained (learned) state judging device.

In the case of one region intended for the state judgment selected by the state judgment region selection unit 204, the state judgment region selection unit 204 outputs the judgment result of the region. In the case of a plurality of regions intended for the state judgment, an identification result is calculated on the basis of, for example, the majority rule of the judgment results and the total of identification scores with respect to the plurality of regions.

In addition, the state judgment may be performed by using one or more images integrated by performing the statistical process with respect to the plurality of regions intended for the state judgment. With this, the more appropriate judgment is enabled. For example, the state judgment may be performed by using an average image. The state judging device may judge the state from the inputted average image. The state judgment may be performed by using the difference image between the plurality of regions intended for the state judgment. The difference image may be, for example, the difference between the maximum value and the minimum value of the pixel at the same position of each of the plurality of regions. The state judging device may judge the state from the inputted difference image and any one of the plurality of images or the average image of them. The difference image is particularly effective for the case where the identification is performed in consideration of the turbidity state of the specimen.

When the state judgment region selection unit 204 outputs the small region, the state judgment unit 205 may judge the LHI for each pixel, that is, on the basis of the color and/or the brightness of each pixel, and calculate the final state judgment result by using the judgment result of each pixel within the small region, the majority rule of the judgment scores, or the statistical value, such as a total sum, an average, and a weighted total sum.

Alternatively, the statistical process for the total sum, the average, the weighted total sum, and the like of the values of the plurality of pixels of the region may be performed to input the value to the state judging device. Alternatively, the image of the small region, that is, the information on the position and the value of the pixel is inputted to the judging device, so that the state judgment result can be calculated with the texture information (the spatial information on the value (the color and/or the brightness) of the pixel) being as the feature amount. With this, the more accurate judgment is enabled. In addition, for the weight of the weighted total sum, for example, a portion of the state judgment properness information calculated by the state judgment properness information calculation unit 203, such as the color properness information may be used.

The output unit 206 will be described.

The output unit 206 outputs the state judgment result of the specimen outputted by the state judgment unit 205 and/or the position information within the image of the region intended for the state judgment outputted by the state judgment region selection unit 204.

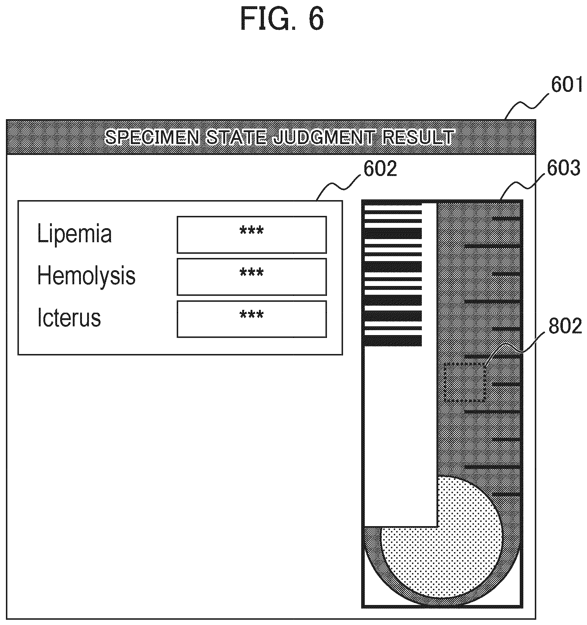

Fig. 6 illustrates an example for the case where the output result is displayed on the screen. A window 601 represents a window presented to the user, a state judgment result display unit 602 represents the specimen state judgment result, and a state judgment intended region display unit 603 represents, as an image, the position within the input image of the region intended for the state judgment. However, the output form is not limited to the example of Fig. 6. For example, the position information of the region intended for the state judgment is not necessarily displayed as the image, but may be displayed by a character string as a start point coordinate and an end point coordinate, or a character string as a set of a start point coordinate, a width, and a height. In addition, the output form is not necessarily displayed for each sample, but may be a form such that the IDs and the names of a plurality of samples, the state judgment result, and the coordinate information (the start point coordinate, the end point coordinate, and the like) of the region intended for the state judgment are listed.

In addition, the output form may be a form in which these displays are recorded into the memory 112 once, and are displayed in any form by an instruction from the user.

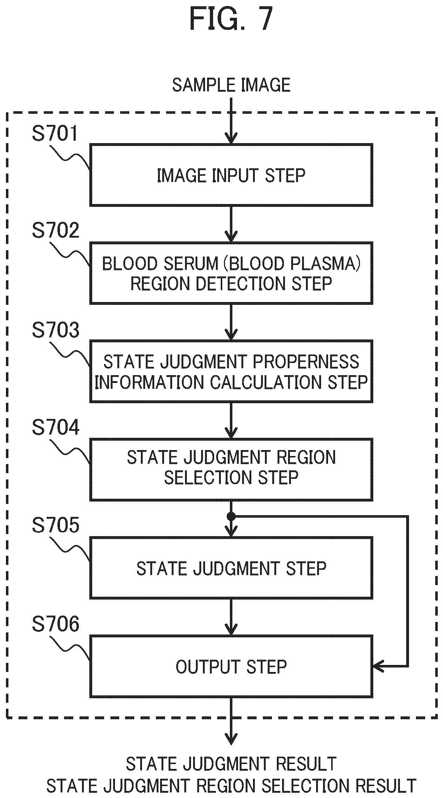

In the above, the detail of the first embodiment has been described for each functional block. However, the embodiment of the present invention is not necessarily required to be configured of the functional block of Fig. 2, and the process that achieves the operation of each functional block should be achieved. Fig. 7 illustrates an example of a process flowchart according to the first embodiment. Each step corresponds to each element of the functional block diagram illustrated in Fig. 2.

The image input step S701 receives the image that is inputted from the interface 110 and is acquired by imaging the sample to be state judged from the side surface of the sample.

The blood serum (blood plasma) region detection step S702 detects the blood serum or blood plasma region from the sample image received in the image input step S701.

The state judgment properness information calculation step S703 calculates the information (state judgment properness information) for evaluating the properness degree with respect to the state judgment from the blood serum or blood plasma region calculated in the blood serum (blood plasma) region detection step S702.

The state judgment region selection step S704 selects one or more partial regions intended for the state judgment from within the blood serum or blood plasma region on the basis of the state judgment properness information calculated in the state judgment properness information calculation step S703.

The state judgment step S705 judges the state (LHI) of the specimen by using the partial region intended for the state judgment selected in the state judgment region selection step S704.

The output step S706 outputs the state judgment result of the specimen calculated in the state judgment step S705 and/or the position information of the partial region intended for the state judgment selected in the state judgment region selection step S704.

According to the specimen state judging apparatus described in the first embodiment, even in the case where the factor that visually affects the blood serum or blood plasma region is included within the image and in the case where the blood serum or blood plasma region is not uniform, the state of the specimen can be judged with high accuracy.

Second Embodiment

A second embodiment describes a specimen analyzing system that judges the state of the specimen by using the specimen state judging apparatus described in the first embodiment and controls the conveying path of the sample according to the state judgment result of the specimen.

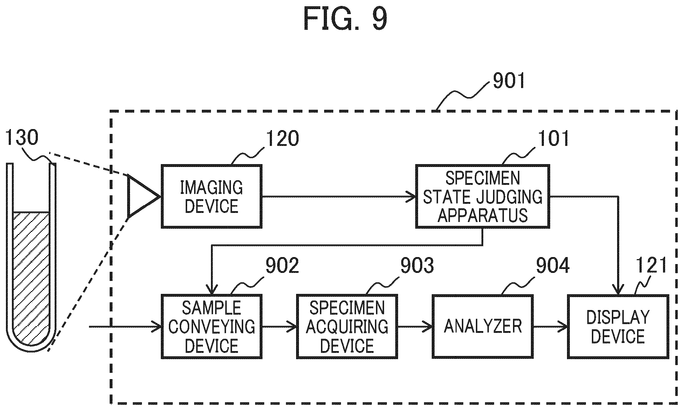

Fig. 9 illustrates a system configuration diagram according to the second embodiment.

A specimen analyzing system 901 includes the imaging device 120 that images the sample 130 from the side surface of the sample 130, the specimen state judging apparatus 101 described in the first embodiment, a sample conveying device 902 conveying the sample 130, a specimen acquiring device 903 that acquires the specimen from the sample conveyed by the sample conveying device 902, an analyzer 904 that analyzes the specimen acquired by the specimen acquiring device 903, and the display device 121 that presents, to the user, the specimen analysis result by the analyzer 904, and the specimen state judgment result by the specimen state judging apparatus 101 and/or the position information of the region intended for the state judgment.

The imaging device 120 is similar to the hardware component of the first embodiment, and the description thereof is thus omitted. Hereinbelow, the sample conveying device 902, the specimen acquiring device 903, the analyzer 904, and the display device 121 will be described.

The sample conveying device 902 is a device for conveying the sample, and is, for example, a belt conveyor that conveys the sample (the test tube containing the specimen) fixed to the holder, and the like. The sample conveying device switches the conveying path of each sample on the basis of the specimen state judgment result of the specimen state judging apparatus 101 with respect to each sample. Specifically, the sample in which the LHI is Negative or the level or the index value of the LHI is judged to be the level or the index value that is the predetermined threshold value or less is conveyed to the specimen acquiring device 903, and conveys the sample other than that to, for example, the temporary storing space. The sample conveyed to the temporary storing space is not analyzed by the analyzer 904, and its process is decided by the user, or the sample is conveyed to the different device.

The specimen acquiring device 903 is a device that acquires the specimen in the sample conveyed by the sample conveying device 902, and is an aspiration nozzle and the like.

The analyzer 904 is a device that performs biochemical analysis and immune analysis by using the reaction solution made by reacting the specimen acquired by the specimen acquiring device 903 with the reagent.

The display device 121 displays the analysis result by the analyzer 904, and the specimen state judgment result by the specimen state judging apparatus 101 and/or the position information of the region intended for the state judgment.

In the case of the sample in which one of the LHI is Positive, this may affect the analysis result. By the above configuration, the sample in which each of the LHI is judged to be Positive or has a high index value by the specimen state judging apparatus 101 is not subjected to the specimen acquirement by the specimen acquiring device 903 and to the analysis by the analyzer 904. With this, the analysis is enabled with respect to only the normal sample, the sample and the specimen containing the LHI can be prevented from wasted, and the analysis time and the reagent can be saved.

<Modification Example>

The method by which the blood serum (blood plasma region image) region detection unit 202 extracts and outputs, as the blood serum or blood plasma region information, the blood plasma or blood plasma region image has been described, but for example, the input image received from the image input unit 201 and rectangular information and mask information representing the blood serum or blood plasma region may be outputted. In addition, without acquiring the entire blood serum or blood plasma region, for example, only the blood serum or blood plasma upper end may be detected, thereby extracting, as the blood serum or blood plasma region, the region having a certain height from the blood serum or blood plasma upper end. In addition, when the region division method is used, at this point in time, the region of the label, the printing, and the like may be judged to, and excepted from the candidate region.

In the state judgment properness information calculation unit 203, the color properness degree information has been described, but for example, when the images (training images and the like) used for constructing the state judging device used in the state judgment unit 205 are present, these images may be used as the calibration data. In this case, it is expected that as the value of the color properness degree information is higher, the reliability degree of the result of the state judging device becomes higher. In addition, when the color is judged by using the color histogram and the like, the judging method intended for each of the blood serum and the blood plasma may be prepared. For example, in the case of the color histogram, the calibration data in which only the blood serum is collected and the calibration data in which only the blood plasma is collected may be prepared, thereby creating the histogram intended for the blood serum and the histogram intended for the blood plasma. In addition, when the color similarity degree and the like is judged, any colorimetric system, such as an RGB, an HSV, and an HLS may be used.

The state judgment region selection unit 204 may select the region intended for the state judgment on the basis of the magnitude of the value of any of state judgment properness information. For example, the pixel or the region in which the color properness degree in the candidate region is maximum may be selected as the region intended for the state judgment. In Fig. 5, the example in which all the pixels are scanned has been described, but when a certain number or more of candidate regions are found, the number of times of scanning may be reduced under some condition by, for example, moving to the next step.

In addition, at least one of the blood serum (blood plasma) region detection unit 202, the state judgment properness information calculation unit 203, or the state judgment unit 205 may perform the blood serum (blood plasma) region detection, the state judgment properness information calculation, the state judgment, and the like by using the result obtained by applying the image process, such as the normalization and correction of the color and the brightness, the filtering, and the retinex with respect to the image to be processed.

As the image process, for example, there is a method by which the characteristic of the illumination is formulated, and on the basis of its characteristic, the color tone and the brightness value within the image are normalized and corrected. The illuminance degree that each pixel receives from the illumination can be formulated on the basis of the distance and the angle with respect to each pixel and the illumination. On the basis of the formulated illuminance degree, for example, the brightness value can be corrected such that the brightness value of the portion having a low illuminance degree can be corrected so as to be the brightness value of the pixel having the highest illuminance degree.

In addition, when a plurality of illuminations are used, the color temperature may be different according to the illumination. When the state of the specimen is judged on the basis of the color, the color is preferably uniform. Accordingly, from the specification of each illumination or the previously measured color temperature, for example, the shift amount of the color phase at each pixel is formulated. By correcting the color phase at each pixel on the basis of the formulated shift amount of the color phase, the change in the color phase due to the difference in the illumination can be reduced.

In addition, the component separation process such as the retinex may be applied. The retinex is a process for separating the component having the constant color or brightness of the object and the component having the color or brightness by the illumination from each other. By extracting the information on the constant color or brightness by the retinex, the correction image in which the difference in the color or the brightness by the illumination at each coordinate is reduced can be acquired.

By using such the image process to perform the blood serum (blood plasma) region detection, the state judgment properness information calculation, the state judgment, and the like, the more accurate detection and judgment are enabled.

It should be noted that the present invention is not limited to the above embodiments, and includes various modification examples. For example, the above embodiments have been described in detail to simply describe the present invention, and are not necessarily limited to include all the described configurations. In addition, part of the configuration of one embodiment can be replaced with the configurations of other embodiments, and in addition, the configuration of the one embodiment can also be added with the configurations of other embodiments. In addition, part of the configuration of each of the embodiments can be subjected to addition, deletion, and replacement with respect to other configurations.

In addition, the above respective configurations, functions, processing units, and the like may be achieved by hardware by, for example, designing part or all of them by, for example, an integrated circuit. In addition, the above respective configurations, functions, and the like may be achieved by software with the processor interpreting and executing the program achieving the respective functions. The information on the program, the table, the file, and the like achieving each function can be placed on a recording device, such as a memory, a hard disk, and an SSD (Solid State Drive), or a recording medium, such as an IC card and an SD card.

In addition, any control lines and information lines that are considered to be required for the description are represented, and for the product, all the control lines and information lines are not necessarily represented. It may be considered that actually, almost all the configurations are mutually connected.

**Claims**

1. A specimen state judging apparatus that judges the state of a specimen stored in the inside of a vessel, comprising:

   an image input unit that receives an image obtained by imaging the vessel storing the specimen from the side surface of the vessel;
   a region detection unit that identifies a blood serum or blood plasma region from within the image;
   a state judgment properness information calculation unit that calculates state judgment properness information as information for evaluating a properness degree intended for the state judgment of the specimen for each partial region of the blood serum or blood plasma region identified by the region detection unit;
   a state judgment region selection unit that selects the partial region suitable for the state judgment of the specimen on the basis of the state judgment properness information;
   a state judgment unit that judges the state of the specimen by using the partial region selected by the state judgment region selection unit; and
   an output unit that outputs the state judgment result of the specimen judged by the state judgment unit and/or position information within the image of the partial region selected by the state judgment region selection unit.

2. The specimen state judging apparatus according to claim 1,
   wherein the state judgment properness information calculation unit calculates, as information included in the state judgment properness information, edge information for the each partial region.

3. The specimen state judging apparatus according to claim 1,
   wherein the state judgment properness information calculation unit calculates, as information included in the state judgment properness information, the properness degree intended for the specimen state judgment of the color or the brightness of each partial region.

4. The specimen state judging apparatus according to claim 1,
   wherein the state judgment properness information calculation unit calculates, as information included in the state judgment properness information, a distance from a previously designated origin point to the partial region.

5. The specimen state judging apparatus according to claim 1,
   wherein at least one of the region detection unit, the state judgment properness information calculation unit, and the state judgment unit applies, to the image, at least one of normalization and correction, filtering, or a component separation process.

6. The specimen state judging apparatus according to claim 1,
   wherein the state judgment unit judges the state of the specimen by using the texture information of the partial region.

7. The specimen state judging apparatus according to claim 1,

   wherein the state judgment region selection unit extracts a plurality of partial regions, and
   wherein the state judgment unit judges the state of the specimen by using the image obtained by integrating the plurality of partial regions by a statistical process.

8. A specimen state judging method by which an apparatus judges the state of a specimen stored in the inside of a vessel, comprising:

   allowing the apparatus to receive an image obtained by imaging the vessel storing the specimen from the side surface of the vessel;
   allowing the apparatus to identify a blood serum or blood plasma region from within the image;
   allowing the apparatus to calculate state judgment properness information as information for evaluating a properness degree intended for the state judgment of the specimen for each partial region of the identified region;
   allowing the apparatus to select the partial region suitable for the state judgment of the specimen on the basis of the state judgment properness information;

allowing the apparatus to judge the state of the specimen by using the selected partial region; and

allowing the apparatus to output the result of the judgment and/or position information within the image of the selected partial region.

**9.** The specimen state judging method according to claim 8,

wherein the apparatus calculates, as information included in the state judgment properness information, edge information for each partial region.

**10.** The specimen state judging method according to claim 8,

wherein the apparatus calculates, as information included in the state judgment properness information, the properness degree intended for the specimen state judgment of the color or the brightness of each partial region.

**11.** The specimen state judging method according to claim 8,

wherein the apparatus calculates, as information included in the state judgment properness information, a distance from a previously designated origin point to the partial region of the blood serum or blood plasma region within the image.

**12.** The specimen state judging method according to claim 8,

wherein the apparatus judges the state of the specimen by using texture information of the partial region.

**13.** The specimen state judging method according to claim 8,

wherein the apparatus extracts a plurality of partial regions, and

wherein the apparatus judges the state of the specimen by using the image obtained by integrating the plurality of partial regions by a statistical process.

**14.** A specimen analyzing system that judges the state of a specimen stored in the inside of a vessel and controls the conveying of the vessel storing the specimen on the basis of the result of the judgment, the specimen analyzing system comprising:

an imaging device that acquires an image obtained by imaging the vessel storing the specimen from the side surface of the vessel;

the specimen state judging apparatus according to any one of claims 1 to 7 that judges the state of the specimen within the image;

a conveying device that controls the conveying path of the vessel storing the specimen on the basis of the judgment result of the state of the specimen by the specimen state judging apparatus;

a specimen acquiring device that acquires the specimen from the vessel conveyed by the conveying device;

an analyzer that analyzes the specimen acquired by the specimen acquiring device; and

a display device that displays the analysis result by the analyzer, and the judgment result by the specimen state judging apparatus, and/or position information within the image of a partial region used for judging the state of the specimen.

# FIG. 1

# FIG. 2

SAMPLE IMAGE                    101

201

IMAGE INPUT UNIT

202

BLOOD SERUM (BLOOD PLASMA)
REGION DETECTION UNIT

203

STATE JUDGMENT PROPERNESS
INFORMATION CALCULATION UNIT

204

STATE JUDGMENT REGION
SELECTION UNIT

205

STATE JUDGMENT UNIT

206

OUTPUT UNIT

STATE JUDGMENT RESULT
STATE JUDGMENT REGION SELECTION RESULT

# FIG. 3A

# FIG. 3B

# FIG. 4A

401

# FIG. 4B

402

# FIG. 5

START

S501
x=0, y=0
GENERATE AND INITIALIZE CANDIDATE MAP

S502
EDGE (x, y) < Th_e — N

Y

S504
COLOR
SIMILARITY DEGREE (x, y) ≥
Th_c — N

Y

S507
ACQUIRE NEXT
COORDINATE x, y

S505
CANDIDATE MAP (x, y) = 1

S503
CANDIDATE MAP (x, y) = 0

S506
N — ARE ALL
PIXELS PROCESSED?

Y

S508
SELECT COORDINATE x', y' IN WHICH
CANDIDATE MAP IS 1 AND ABSOLUTE
VALUE DIFFERENCE BETWEEN DISTANCE
FROM UPPER END AND DISTANCE
FROM LOWER END IS MINIMUM

S509
OUTPUT x', y'

END

# FIG. 6

601

SPECIMEN STATE JUDGMENT RESULT

602

603

Lipemia    ***

Hemolysis  ***

Icterus    ***

802

# FIG. 7

SAMPLE IMAGE

S701

IMAGE INPUT STEP

S702

BLOOD SERUM (BLOOD PLASMA)
REGION DETECTION STEP

S703

STATE JUDGMENT PROPERNESS
INFORMATION CALCULATION STEP

S704

STATE JUDGMENT REGION
SELECTION STEP

S705

STATE JUDGMENT STEP

S706

OUTPUT STEP

STATE JUDGMENT RESULT
STATE JUDGMENT REGION SELECTION RESULT

FIG. 8

## FIG. 9

# FIG. 10

## INTERNATIONAL SEARCH REPORT

| International application No. |
|---|
| **PCT/JP2024/030482** |

**A.    CLASSIFICATION OF SUBJECT MATTER**

*G01N 33/49*(2006.01)i; *G01N 35/00*(2006.01)i
FI:    G01N33/49 K; G01N35/00 A

According to International Patent Classification (IPC) or to both national classification and IPC

**B.    FIELDS SEARCHED**

Minimum documentation searched (classification system followed by classification symbols)

G01N33/49; G01N35/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| X | JP 2019-505802 A (SIEMENS HEALTHCARE DIAGNOSTICS INC.) 28 February 2019 (2019-02-28) <br> paragraphs [0019]-[0021], [0023]-[0025], [0027], [0048], [0050]-[0051], [0084]-[0088], [0103]-[0104], [0111] | 1, 5, 7-8, 13-14 |
| Y |  | 2-3, 6, 9-10, 12 |
| A |  | 4, 11 |
| Y | WO 2021/205737 A1 (HITACHI HIGH-TECH CORPORATION) 14 October 2021 (2021-10-14) <br> paragraph [0076] | 2-3, 6, 9-10, 12 |
| Y | JP 2020-516894 A (SIEMENS HEALTHCARE DIAGNOSTICS INC.) 11 June 2020 (2020-06-11) <br> paragraph [0127] | 2-3, 6, 9-10, 12 |

✓ Further documents are listed in the continuation of Box C.          ✓ See patent family annex.

| | |
|---|---|
| *    Special categories of cited documents: <br> "A"   document defining the general state of the art which is not considered to be of particular relevance <br> "D"   document cited by the applicant in the international application <br> "E"   earlier application or patent but published on or after the international filing date <br> "L"   document which may throw doubts on priority claim(s) or which is cited to establish the publication date of another citation or other special reason (as specified) <br> "O"   document referring to an oral disclosure, use, exhibition or other means <br> "P"   document published prior to the international filing date but later than the priority date claimed | "T"   later document published after the international filing date or priority date and not in conflict with the application but cited to understand the principle or theory underlying the invention <br> "X"   document of particular relevance; the claimed invention cannot be considered novel or cannot be considered to involve an inventive step when the document is taken alone <br> "Y"   document of particular relevance; the claimed invention cannot be considered to involve an inventive step when the document is combined with one or more other such documents, such combination being obvious to a person skilled in the art <br> "&"   document member of the same patent family |

| Date of the actual completion of the international search | Date of mailing of the international search report |
|---|---|
| **28 October 2024** | **12 November 2024** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (July 2022)

# EP 4 782 834 A1

## INTERNATIONAL SEARCH REPORT

International application No.

**PCT/JP2024/030482**

### C. DOCUMENTS CONSIDERED TO BE RELEVANT

| Category* | Citation of document, with indication, where appropriate, of the relevant passages | Relevant to claim No. |
|---|---|---|
| A | JP 2016-008927 A (HITACHI HIGH-TECHNOLOGIES CORPORATION) 18 January 2016 (2016-01-18) <br> paragraphs [0022], [0137] | 1-14 |
| A | JP 2016-223914 A (HITACHI HIGH-TECHNOLOGIES CORPORATION) 28 December 2016 (2016-12-28) <br> paragraph [0014] | 1-14 |
| A | WO 2020/153177 A1 (HITACHI HIGH-TECH CORPORATION) 30 July 2020 (2020-07-30) <br> paragraph [0009] | 1-14 |
| A | WO 2015/002218 A1 (HITACHI HIGH-TECHNOLOGIES CORPORATION) 08 January 2015 (2015-01-08) <br> paragraph [0010] | 1-14 |
| A | JP 2020-516885 A (SIEMENS HEALTHCARE DIAGNOSTICS INC.) 11 June 2020 (2020-06-11) <br> paragraphs [0015], [0121] | 1-14 |
| A | JP 2018-511787 A (SIEMENS HEALTHCARE DIAGNOSTICS INC.) 26 April 2018 (2018-04-26) <br> paragraphs [0017], [0019], [0071] | 1-14 |

Form PCT/ISA/210 (second sheet) (July 2022)

## INTERNATIONAL SEARCH REPORT
### Information on patent family members

| | International application No. |
|---|---|
| | **PCT/JP2024/030482** |

| Patent document cited in search report | | | Publication date (day/month/year) | Patent family member(s) | | | Publication date (day/month/year) |
|---|---|---|---|---|---|---|---|
| JP | 2019-505802 | A | 28 February 2019 | US paragraphs [0032]-[0034], [0036]-[0038], [0040], [0061], [0063]-[0064], [0097]-[0101], [0116]-[0117], [0124] | 2018/0372648 | A1 | |
| | | | | WO | 2017/132171 | A1 | |
| | | | | CN | 108474733 | A | |
| WO | 2021/205737 | A1 | 14 October 2021 | US paragraph [0094] | 2023/0097384 | A1 | |
| | | | | EP | 4134677 | A1 | |
| | | | | CN | 115280158 | A | |
| JP | 2020-516894 | A | 11 June 2020 | US paragraph [0158] | 2020/0158745 | A1 | |
| | | | | WO | 2018/188023 | A1 | |
| | | | | CN | 110573883 | A | |
| JP | 2016-008927 | A | 18 January 2016 | (Family: none) | | | |
| JP | 2016-223914 | A | 28 December 2016 | (Family: none) | | | |
| WO | 2020/153177 | A1 | 30 July 2020 | US paragraph [0011] | 2022/0082492 | A1 | |
| | | | | EP | 3916375 | A1 | |
| | | | | CN | 113302472 | A | |
| WO | 2015/002218 | A1 | 08 January 2015 | US paragraph [0009] | 2016/0109350 | A1 | |
| | | | | EP | 3018482 | A1 | |
| | | | | CN | 105324671 | A | |
| JP | 2020-516885 | A | 11 June 2020 | US paragraphs [0014], [0139] | 2020/0151878 | A1 | |
| | | | | WO | 2018/191295 | A1 | |
| | | | | CN | 110520737 | A | |
| JP | 2018-511787 | A | 26 April 2018 | US paragraphs [0016], [0018], [0078] | 2018/0045654 | A1 | |
| | | | | WO | 2016/133900 | A1 | |

Form PCT/ISA/210 (patent family annex) (July 2022)

**EP 4 782 834 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023158397 A

- JP 2020519853 W